# EUROPEAN PATENT SPECIFICATION

(11) **EP 3 187 189 B1**
(45) Date of publication and mention of the grant of the patent: **18.11.2020**
(21) Application number: 15836301.0
(22) Date of filing: 28.08.2015
(51) Int. Cl.: A61K 36/9066, A61K 36/82, A61K 31/05, A61P 3/04

(54) **COMPOSITION FOR USE IN TREATING OBESITY AND USE OF SAID COMPOSITION FOR REDUCING BODY WEIGHT AND FAT**
ZUSAMMENSETZUNG ZUR VERWENDUNG IN DER BEHANDLUNG VON ADIPOSITAS UND VERWENDUNG DER ZUSAMMENSETZUNG ZUR REDUZIERUNG DES KÖRPERGEWICHTS UND KÖRPERFETTS
COMPOSITION POUR L'UTILISATION DANS LE TRAITEMENT DE L'OBESITE ET UTILISATION DE LADITE COMPOSITION POUR RÉDUIRE LA MASSE ET GRAISSE CORPORELLE

(30) Priority: 28.08.2014 US 201462042955 P
(43) Date of publication of application: 05.07.2017
(73) Proprietor: Caliway Biopharmaceuticals Co. Ltd., New Taipei City 221 (TW)
(72) Inventor: LING, Yu-Fang, New Taipei City 221 (TW)
(74) Representative: Haseltine Lake Kempner LLP
(86) International application number: PCT/CN2015/088338
(87) International publication number: WO 2016/029868

(56) References cited:
- WO-A1-2014/028607
- CN-A- 101 095 665
- CN-A- 101 632 655
- CN-A- 102 357 226
- US-A1- 2006 172 012
- US-A1- 2009 239 943
- US-A1- 2014 141 082
- WANG SHU ET AL: "Novel insights of dietary polyphenols and obesity", THE JOURNAL OF NUTRITIONAL BIOCHEMISTRY, vol. 25, no. 1, 5 December 2013 (2013-12-05), pages 1-18, XP028794865, ISSN: 0955-2863, DOI: 10.1016/J.JNUTBIO.2013.09.001
- DONG-HU ZHOU ET AL: "Combination of Low Concentration of (-)-Epigallocatechin Gallate (EGCG) and Curcumin Strongly Suppresses the Growth of Non-Small Cell Lung Cancer in Vitro and in Vivo through Causing Cell Cycle Arrest", INTERNATIONAL JOURNAL OF MOLECULAR SCIENCES, vol. 14, no. 6, 5 June 2013 (2013-06-05), pages 12023-12036, XP055325831, DOI: 10.3390/ijms140612023
- DATABASE GNPD [Online] MINTEL; August 2009 (2009-08), anonymous: "Slimming Tea", XP002778254, retrieved from www.gnpd.com Database accession no. 1157255
- DATABASE GNPD [Online] MINTEL; June 2014 (2014-06), anonymous: "Whole Body Healthy Inflammation Response Dietary Supplement", XP002778255, retrieved from www.gnpd.com Database accession no. 2512819

## Description

### Field of the Invention

The present invention relates to a composition, and more particularly to a composition comprising a plant extract with a specific weight ratio of green tea extract to turmeric extract. The present invention relates to an application of the composition, and more particularly the application of the composition in preparing a pharmaceutical composition for reducing weight and body fat. The present invention relates to a pharmaceutical composition comprising the composition, and more particularly to a pharmaceutical composition which can be used for reducing weight and reducing body fat. The present invention relates to an application of the pharmaceutical composition, and more particularly to the application in reducing weight and body fat by using an effective dosage of the pharmaceutical composition.

### Background of the Invention

According to the definition of obesity of World Health Organization (WHO), the body mass index (BMI) greater than 25 is defined as overweight and BMI greater than 30 is defined as obesity. Statistical data shows the population of overweight and obesity around the world is over 2.7 billion in 2014, wherein approximately 13% population were obese. The chance of these obese people who might suffer from related diseases such as, cardiovascular diseases, hyperlipidemia, diabetes, and cancers are drastically increased than average people. A research report of the WHO also indicated that among the diseases which causes risks of mortality around the world, overweight and obesity ranked 6^{th}. According to research data, at least more than 3.4 million adults die of chronic diseases caused by overweight or obesity in 2013, wherein 44% of the medical burden of diabetes and 23% of the medical burden of ischemic heart disease are attributable to obesity. Many data also showed that the age of obese people is in a gradually falling trend. According to WHO data, there are approximately 40 million children under the age of five are overweight worldwide in 2011. According to a report published by the Johns Hopkins University Bloomberg School of Public Health in 2007, indicating that approximately 75% of adults in the USA would be overweight, wherein 41% of the population thereof would be classified as obese in 2015. With the rising of developing countries, the global population of obesity will rapidly increase and become one of the major prevalent diseases. The Centers for Disease Control and Prevention (CDC) of the USA indicated that the population of obese adults in the USA is more than 72 million, and 40 % of global obese population is in the Asia-Pacific region. The percentage of overweight and obese adults in China was drastically increased from 25% in 2002 to 38.5% in 2010; it predicts that, in 2015, 50% to 57% of the population in China will be overweight.

Obesity is a health problem highly concerned worldwide, and studies shows that the causes of obesity are highly complex with multiple factors involved. More and more evidences also show that obesity is an internal metabolic disorder disease which is not a simple problem that can be improve by self-control, but a complex symptom related to internal appetite regulation and energy metabolism. Obesity not only increases mortality rate and causes huge medical burden, but also affects the quality of life to mankind. Though the cause of obesity is not completely identified, it is believed to be related to genetics, metabolism, biochemistry, culture, and spiritual-social factors. Research shows many causes of death are correlated with obesity including cancers, cardiovascular diseases, cerebrovascular diseases, diabetes, chronic lower respiratory diseases, chronic hepatic disease and liver cirrhosis, hypertensive diseases, renal disease, etc., indicating that the problem of obesity has become a highly concerned issue globally. In recent years, the prevalence of obesity has risen higher and higher, metabolic syndromes caused by metabolic abnormalities such as, high blood pressure (hypertension), high blood sugar (hyperglycemia), insulin resistance, and dyslipidemia, would gradually appeared and are accompanied by obesity, which would easily leads to diseases such as, diabetes, cardiovascular diseases, atherosclerosis, cerebrovascular disease, and cancer, which cause stroke, myocardial infarction, and even death.

The mechanisms of current drugs for losing weight can be divided into two categories, which is appetite suppression and blocking part of the intestinal absorption of dietary fat respectively; wherein appetite suppression is the main mechanism of commercial weight loss drugs on the market in the past and nowadays; this type of drugs comprising Sibutramine (Reductil®), Lorcaserin, Qsymia®, and Contrave, etc, which have more severe side effects and a certain degree of cardiovascular diseases risk. Take the weight loss drug Sibutramine (Reductil®), which has been discontinued, for example, its market share was once as high as 70 percent. Sibutramine (Reductil®) achieves the weight loss effect through dual effect which is affecting the central nervous system to increase satiety and promoting the increase of metabolic rate in the periphery of the body. Sibutramine is a reuptake inhibitor of noradrenaline and serotonin which increases satiety to suppress appetite to achieve the purpose of weight loss, wherein the increase in satiety is achieved by the mechanism that serotonin and noradrenaline, the uptake thereof are inhibited, interact withα1-adrenoceptors, β1-adrenoceptors and 5-HT2 receptor subtypes. The effect of Sibutramine on the central nervous system may cause the increase in blood pressure and increase heart rate, and Sibutramine was proved to increase the risks of causing cardiovascular disease in recent years; therefore, the weight loss drugs containing Sibutramine ingredient were recalled from the markets of the EU, the United States, Australia, and Taiwan in 2010.

The weight loss drug which blocks part of the intestinal absorption of dietary fat is Orlistat; it is the only legitimate weight loss drug ingredient for long-term use in most countries. It is a specific, reversible gastrointestinal lipase inhibitor functioning in the stomach and small intestine. Orlistat forms a covalent bond with the serine in the active site in the lipase secreted from stomach and pancreas to inactivate the lipase and thus, the lipase cannot hydrolyze the fat in the form of triglyceride in the diet into absorbable free fatty acids and monoglycerides. Due to the undigested triglycerides are unabsorbable, it will be got rid of the body directly. By means of the inhibition of lipase secreted from pancreatic and intestine, the intestinal absorption of dietary fat could be reduced up to 25% to 30%. Due to the main mechanism of Orlistat is blocking fat absorption, gastrointestinal side effects such as oily stool, increased number of bowel movements, flatulence, etc. may occur during medication intake, and it would interfere with fat-soluble vitamin absorption; there are also some cases of severe side effects such as liver damage, and gallstones, etc., in foreign countries.

Massive demand and high profits in weight loss drugs have drawn pharmaceutical companies to invest in its research. However, the safety of weight loss drugs is a challenging issue; severe side effects and the risk of cardiovascular disease caused the FDA to stop approving weight loss drugs for years before 2012, and many companies' stock price has been beaten; as a result causing inactivity of the pharmaceutical market. In 2012, FDA finally approved another four new weight loss drugs one after another, Belviq, Qsymia®, Contrave® and Saxenda® respectively, and it was expected to activate the market of weight loss drugs again.

The main ingredients of Qsymia and Belviq are phentermine-topiramate and lorcaserin respectively; the mechanism of Qsymia and Belvig is mainly increasing satiety and suppressing appetite to achieve the purposes of weight loss. Qysmia contains two old drug ingredients, phentermine and topiramate, wherein phentermine is classified as a central nervous system stimulant, its main mechanism of appetite suppression is making hypothalamus stimulate adrenal gland to secret norepinephrine (noradrenaline); the mechanism of topiramate is to increase the activity of the neurotransmitter GABA, block sodium ion channel activity, compete with glutamine for glutamine receptor, and inhibit carbonic anhydrase to achieve the effect on suppressing appetite and increasing satiety.

However, as early as in 1997, 24 subjects, after taking weight loss drugs Fen-Phen (fenfluramine / dexfenfluramine-phentermine) contained phentermine, had heart valve problems; hence caused the recall of fenfluramine and dexfenfluramine from the market requested by the U.S. FDA. Due to Phentermine had caused severe cardiovascular risk in the past, it is still banned in many countries. Topiramate, another ingredient, is a drug which had been approved to treat epilepsy. The side effects, of which people are already aware, of Phentermine-topiramate drug include tingling and numbness of hands and feet, dizziness, dysgeusia, insomnia, constipation, and dry mouth.

Lorcaserin is a 5-HT2C receptor activator, which active pro-opiomelanocortin neurons (POMC neurons) to produce α-MSH (melanocyte stimulating hormone), and further induce satiety, suppress appetite and reduce dietary energy intake. Lorcaserin interacts with 5-HT2C receptors with its high selectivity, the interactions of Lorcaserin with 5-HT2A or 5-HT2B receptors are small; hence it is considered, from its mechanism, that it can reduce the above-mentioned risk of severe cardiovascular diseases. The related side effects of Lorcaserin comprise heart valve damage, headache, nausea, fatigue, and urinary tract infections; therefore, FDA still required the industry to conduct follow-up clinical monitoring; meanwhile, if the body weight does not reduce after Lorcaserin is taken for three months consecutively, the medication needs to be ceased immediately.

Contrave® is a dopamine and norepinephrine reuptake inhibitor, which acts in the central nervous system, and could suppress appetite. The side effects of Contrave® are suicidal tendency, nausea, constipation, headache, vomiting, and dizziness. Saxenda® is a weight-loss drug administered by subcutaneous injection, and the main mechanism of Saxenda® is reducing the rate of gastric emptying to increase satiety and thus achieving the purpose of weight loss; the side effects are nausea, hypoglycemia (low blood sugar), diarrhea, constipation, vomiting, headache, loss of appetite, etc. Overall, the risk of cardiovascular disease and the safety of long-term use of new weight loss drugs, currently on the market, remained to be monitored for longer period; therefore, they are not suitable for patients who have suffered from cardiovascular diseases to take, and numerous side effects and safety concerns remain; especially phentermine which is contained in Qsymia® has caused severe cardiovascular diseases in the past, which made Qsymia® remains to be forbidden in Taiwan and many countries.

The main concern of the development of weight loss drug applications is the safety issue such as cardiovascular risk or mental safety concerns; the side effects such as dizziness, insomnia, palpitations, constipation, etc. and cardiovascular risks have let users could not keep their mind on relax to use the current weight loss drug for long-term. As the currently approved weight loss drugs have severe side effects, poor tolerance, and cardiovascular risk, the overall market of weight loss drugs has not kept pace with the global obese population and the demand for weight loss. Five among the ten approved weight loss drugs from 1957 to 2014 which all reduce weight through the mechanism involving appetite suppression were recalled from the market because of their cardiovascular risks or psychiatric safety concern, including Sibutramine (trade name Reductil®) which launched in 2002 and instantly had 70% of the market share after its launch.

Shu Wang et al. (2014) Novel insights of dietary polyphenols and obesity, J. Nut. Biochem., 25, 1-18, describes potential roles of dietary polyphenols in obesity.

Therefore, the current weight loss drugs remain to have different degree of cardiovascular risks and safety concerns; the market demands a weight loss drugs that is safer, with low side effects, without cardiovascular risk concerns, could effectively reduce weight and body fat, and reduce cardiovascular risk at the same time.

### SUMMARY OF THE INVENTION

The invention is defined as set forth in the claims. All other embodiments are described for illustrative purposes only, and any statement that they would also be part of the invention or other, similar statements, have to be seen in the context of the application as filed and do not alter the scope of the claims.

The present disclosure provides a plant extract composition for reducing body weight and body fat. The plant extract composition comprises a green tea extract and a turmeric extract, and the weight percentages of green tea extract and turmeric extract are respectively 30 % to 75 % and 20 % to 55 % based on the total weight of the plant extract composition.

The present invention provides a composition comprising a green tea extract and a turmeric extract for use in a method of treating obesity, wherein the weight percentages of green tea extract and turmeric extract are respectively 30 % to 75 % and 20 % to 55 % based on the total weight of the composition, wherein the green tea extract comprises 75% to 100% catechins and the turmeric extract comprises 80% to 100% curcumin, and wherein the method comprises administration of the composition to a subject such that body weight or body fat of the subject is reduced.

The present invention also provides the use of a composition for non-therapeutic reduction of body weight or body fat of a subject, wherein the composition comprises a green tea extract and a turmeric extract, the weight percentages of green tea extract and turmeric extract are respectively 30 % to 75 % and 20 % to 55 % based on the total weight of the composition, wherein the green tea extract comprises 75% to 100% catechins and the turmeric extract comprises 80% to 100% curcumin, and wherein the composition is to be administered to the subject such that body weight or body fat of the subject is reduced.

In a preferred embodiment, the plant extract composition of the present invention further comprises resveratrol, wherein the percentage of the resveratrol is more than 0% and is less than or equal to 30 % based on the total weight of the composition.

The plant extract composition of the present invention could effectively reduce weight and body fat. Wherein, what is worth noticing is that the animal experiment indicated that the treatment of administering resveratrol alone has no significant effect on reducing weight and body fat, nor does the treatment of administering turmeric extract alone in animal experiments has significant effects; this is consistent with the previous research result. In the present invention, it was observed that the plant extract composition of the present invention could achieve the effect of significantly reduce weight and body fat not only under the condition that "the plant extract composition of the present invention was administered during obesity induction", but further discovered that it could significantly reduced body weight and body fat in mice even under the condition that "the plant extract composition of the present invention was administered after the obesity had been induced". Compared to that of the commercially available weight loss drug Orlistat, the effect of the plant extract composition of the present invention on reducing weight and body fat is significantly better than Orlistat (p <0.001). This model in which obesity induction is prior to administration is more difficult to reduce weight and body fat than the conventional and commonly seen animal model in which administration is performed during obesity induction, but this model much more resembles the situation of clinically treating the overweight and obese people; under this circumstance, the significant effects of the plant extract composition of the present invention on reducing weight and body fat could still be achieved, and the effects are better than those of commercial weight loss drugs or single plant extract, which shows that the composition of the present invention is not easily achievable.

According to the present invention, the term "turmeric extract" as used herein is an extract comprises curcumin, wherein the concentration of curcumin in the turmeric extract is from 80% to 100%. The term "green tea extract" as used herein is an extract comprises catechins, and the concentration of catechins in the green tea extract is from 75% to 100%.

Also disclosed is a method for preparing the plant extract composition comprising green tea extract and turmeric extract, comprising mixing a plant extract composition comprising green tea extract and turmeric extract with a pharmaceutically acceptable salt composition, a pharmaceutically acceptable stabilizer, or a pharmaceutically acceptable excipient to produce capsules, tablets, coated tablets or injection fluids.

Preferably, the method further comprises a step of adding resveratrol to form a plant extract composition comprising green tea extract, turmeric extract, and resveratrol.

Preferably, the stabilizers include, but are not limited to, xylitol, sorbitol, polydextrose, isomalt, and D-glucose.

Also disclosed is an application of the plant extract composition used for reducing body weight and body fat in the manufacture of a pharmaceutical composition for reducing body weight and body fat.

The present disclosure further provides a pharmaceutical composition used for reducing weight and body fat, comprising an effective amount of the plant extract composition which is used for reducing weight and body fat and a pharmaceutically acceptable excipient. In embodiments of the present invention, the composition is a pharmaceutical composition and further comprises a pharmaceutically acceptable excipient.

In a preferred embodiment, the pharmaceutical composition further comprises an effective amount of resveratrol for reducing body weight and body fat.

According to the present invention, the term "a pharmaceutically acceptable excipient" as used herein includes, but is not limited to, disintegrant, binder, filler, lubricant, suspending agent, solubilizer, and glidant. The quantity of excipient required will depend upon the quantity of the active ingredient, and one excipient can perform one or more function.

Preferably, the disintegrants include, but are not limited to, agar, alginic acid, calcium carbonate, carboxymethylcellulose, cellulose, clays, colloidal silica, croscarmellose sodium, cross-linked povidone, gum, magnesium aluminum silicate, methyl cellulose, polacrilin potassium, sodium alginate, low substituted hydroxypropyl cellulose, crosslinked polyvinylpyrrolidone hydroxypropylcellulose, sodium starch glycolate, or starch.

Preferably, the binders include, but are not limited to, microcrystalline cellulose, hydroxymethyl cellulose, hydroxypropyl cellulose, or polyvinyl pyrrolidone.

Preferably, the fillers include, but are not limited to, calcium carbonate, calcium phosphate, dibasic calcium phosphate, tribasic calcium sulfate, calcium carboxymethylcellulose, cellulose, dextrin, salt, dextrin, dextrose, fructose, lactitol, lactose, carbonate, magnesium oxide, maltitol, maltodextrin, maltose, sorbitol, starch, sucrose, sugar, or xylitol.

Preferably, the lubricants include, but are not limited to, agar, calcium stearate, ethyl oleate, ethyl laurate, glycerin, glyceryl palmitostearate, hydrogenated vegetable oil, magnesium oxide, magnesium stearate, mannitol, poloxamer, ethylene glycol, sodium benzoate, sodium lauryl sulfate, sodium stearoyl acid, sorbitol, stearic acid, talc or zinc stearate.

Preferably, the suspending agents include, but are not limited to, mannitol, carboxymethyl cellulose (CMC), or CMC-Na.

Preferably, the solubilizers include, but are not limited to, hydroxypropyl-beta-cyclodextrin, tween 80, castor oil or polyethylene glycol (PEG).

Preferably, the glidants include, but are not limited to materials such as magnesium stearate, silicon dioxide, magnesium trisilicate, powdered cellulose, starch, talc, tribasic calcium phosphate, calcium silicate, magnesium silicate, colloidal silica or silicon hydrogel.

The pharmaceutical composition of the present invention can exist in multiple forms, including, but not limited to, liquid, semi-solid and solid pharmaceutical forms, such as liquid solution (e.g. injectable and infusible solution), dispersions, suspensions, tablets, pills, powders, liposomes or suppositories. The preferred form depends on the expected mode of administration and therapeutic application. Preferably, the pharmaceutical composition of the present invention is orally administrable form or the form of infusion solutions. In an embodiment of the present invention, the pharmaceutical composition used for reducing weight and body fat comprising an effective amount of a green tea extract and turmeric extract is orally administered. The suitable and preferred dosage forms of the plant extract composition according to the present invention are pill, granules, coated tablets, capsules, tablets and other solid oral dosage forms which are also covered within the scope of the present invention.

Also provided is an application of the pharmaceutical composition in reducing weight and body fat, wherein it achieves the effect of reducing weight and body fat through administering an effective amount of a pharmaceutical composition comprising a plant extract composition which contains green tea extract and turmeric extract to a subject. In embodiments of the present invention, the subject is a human or animal.

Preferably, the administration method is oral administration or injection.

Preferably, the effective amount of the pharmaceutical composition to be administered to the subject every day is 1.8 mg/kg to 145 mg/kg; the subject is a human or an animal; human is preferred.

Preferably, the effective amount of the pharmaceutical composition to be administered to the subject every day is 5.4 mg/kg to 70 mg/kg; the subject is a human or an animal; human is preferred.

According to the present invention, the "effective amount (effective dose)" for different subjects could be calculated according to the Table 1 in "estimating the maximum safe starting dose in initial clinical trials for therapeutics in adult healthy volunteers" published by the Food and Drug Administration (FDA).

According to the present invention, the term "reducing weight and body fat" as used herein refer to both of the body weight and body fat are less than the obese control group after administration of an effective amount of the composition comprising green tea extract and turmeric extract or further comprising resveratrol. The amount of body fat reduction can be detected by administering a specific range of the dosage of the composition comprising a green tea extract and a turmeric extract or further comprising resveratrol, and measuring the changes in the mass of the epididymis fat, perinephric fat, mesenteric fat, groin fat and fat outside the peritoneal cavity in a specific period.

All of the constituents of the plant extract composition of the present invention are extracted from plants, and the results of the experiment indicated that the plant extract composition of the present invention neither affects appetite or the amount of food intake, nor affects other serum biochemical safety index; therefore, it is safer; compared to other weight loss drugs on the market, it is much safer and without significant side effects. Furthermore, compared to the current techniques of weight loss drugs achieving weight reduction effect through the method of inhibiting appetite or blocking intestinal dietary fat absorption to reduce calorie absorption, the plant extract composition of the present invention could not only reduce weight, but also could effectively inhibit adipocyte proliferation in vivo, and simultaneously increases the metabolism of fat and the energy expenditure in vivo. The plant extract composition of the present invention targets the fundamental cause of obesity to improve obesity fundamentally, so as to reduce the common issue of weight regain after weight loss and improve the cardiovascular risk indicator including blood lipids and blood sugar to reduce cardiovascular risks.

Therefore, the plant extract composition of the present invention which targeted the current global issues of obese and overweight provides a solution which is safer and can effectively reduce weight and body fat, and it can be applied to the usage of related pharmaceutical composition or health food.

### BRIEF DESCRIPTION OF THE DRAWINGS

Fig. 1 is a bar chart presenting the effect on preadipocyte growth inhibition in each group tested by MTT assay in the present invention;
Fig. 2 is a bar chart presenting the effect on differentiating adipocyte growth inhibition in each group tested by MTT assay in the present invention;
Fig. 3A is a bar chart presenting the change in weight gain of mice in each group obtained under a model in which administration is performed during obesity induction in the present invention.
Fig. 3B is a bar chart presenting the change in fat mass of visceral fat, subcutaneous fat, and total fat mass of mice in each group obtained under a model in which administration is performed during obesity induction in the present invention.
Fig. 4A is a bar chart presenting the change in weight gain of mice in each group obtained under a model in which obesity induction is prior to administration.
Fig. 4B is a bar chart presenting the change in fat mass of visceral fat, subcutaneous fat, and total fat mass of mice in each group obtained under a model in which obesity induction is prior to administration.
Fig. 5A is a run chart presenting the change of weight gain of rat in each group obtained under a model in which administration is performed during obesity induction in the present invention.
Fig. 5B is a bar chart presenting the change of fat mass of visceral fat of rat in each group obtained under a model in which administration is performed during obesity induction in the present invention.

### DETAILED DESCRIPTION OF THE INVENTION

The technical means, accompanied by the drawings and the preferred embodiments of the present invention, taken to achieve the projected invention purpose could be further elaborated below.

### Example 1 Preadipocyte growth inhibition test

In this embodiment, 3T3-L1 preadipocytes were incubated at a density of 1x10⁴ cells/well in 96-well plates. With the exception of the control group (DMSO solvent control group), 50 ppm of resveratrol, 50 ppm of turmeric extract, 80 ppm of green tea extract and 100 ppm of the plant extract composition ME008A, ME008D, ME001, MEOOC1, and MEOOD1 were added into different well respectively; there were 9 groups in the experiment, and three replicates were performed in each group. After administration process and 48 hours incubation, photos were taken to record the cell's growth condition; the growth inhibitory effect of respective experimental matters on 3T3-L1 preadipocytes was analyzed by MTT assay. Wherein the control group is the DMSO solvent control group, the plant extract composition ME008A contains 50 wt% green tea extract, 25 wt% green coffee bean extract, and 25 wt% resveratrol. The composition ME008D contains 40 wt% green tea extract, 45 wt% green coffee bean extract, and 15 wt% resveratrol. The composition ME001 contains 60 wt% green tea extract, 10 wt% turmeric extract, and 30 wt% resveratrol. The composition ME00C1 contains 40 wt% green tea extract, 50 wt% turmeric extract, and 10 wt% resveratrol. The composition ME00D1 contains 75 wt% green tea extract and 25 wt% turmeric extract. Data of each group are presented in Mean ± SD. The letters a, b, c, d, e, f, and g represent the results of the statistics; the different letters represent statistical difference among the groups (*p*<0.05), and the identical letter represents no statistical difference among the groups (*p*>0.05).

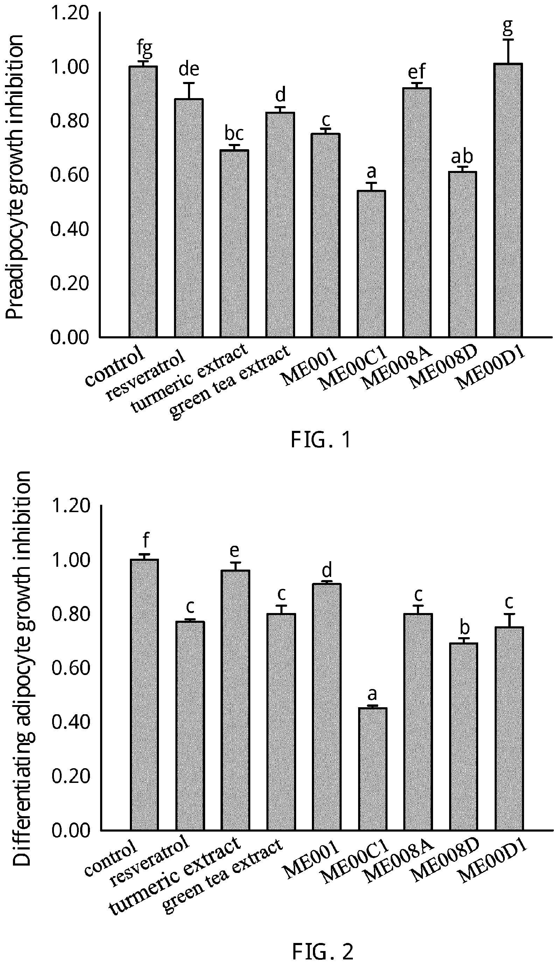

Results are shown in Fig. 1, as compared to the control group, the plant extract composition ME00C1 of the present invention and ME001 and ME008D could all significantly inhibit the cell growth of preadipocytes (*p*<0.05); ME00C1 had the best inhibitory effect on preadipocyte growth among all of the plant extract compositions of the present disclosure and invention (*p*<0.05). The inhibitory effect of the composition ME00C1 on preadipocyte growth was significantly greater than that of the resveratrol, the turmeric extract, or the green tea extract administered alone(*p*<0.05).

### Example 2 Differentiating adipocyte growth inhibition test

In this embodiment, 3T3-L1 cells were incubated at a density of 1x10⁵ cells/well in 12-well plates. The medium was replaced with the medium containing 5 µg/ml insulin differentiation agent, 1 µM dexamethasone, and 0.5 mM 3-isobutyl-1-methylxanthine on the fourth day of incubation to induce adipocyte differentiation. With the exception of the control group (DMSO solvent control group), 50 ppm of resveratrol, 50 ppm of turmeric extract, 80 ppm of green tea extract, and 100 ppm of the plant extract composition ME008A, ME008D, and ME001 of the present disclosure, and ME00C1 and ME00D1 of the present invention were added into different well respectively; there were 9 groups in the experiment, and three replicates were performed in each group. After administration process and 48 hours incubation, photos were taken to record the cell's growth condition; the inhibitory effect of respective experimental matters on differentiating adipocytes was analyzed by MTT assay. Data of each group are presented in Mean ± SD. The letters a, b, c, d, e, and f represent the results of the statistics; the different letters represent statistical difference among the groups (p<0.05), and the identical letter represents no statistical difference among the groups (p>0.05).

Results are shown in Fig. 2, as compared to the control group, all of the plant extract compositions of the present invention could inhibit the cell growth of differentiating adipocyte significantly; ME00C1 had the best inhibitory effect on differentiating adipocyte growth among all of the plant extract composition of the present disclosure and invention. The inhibitory effect of the composition ME00C1 on differentiating adipocyte growth was significantly greater than that of the resveratrol, the turmeric extract, or the green tea extract administered alone *(p<0.05).*

### Example 3 Animal assay (I) (Administration during obesity induction)

In this embodiment, 8-week-old female B6 mice were used. There were five groups in this experiment which were a normal control group, an obese control group, a resveratrol group (61.5 mg/kg B.W.), a green tea extract group (123 mg/kg B.W.), and plant extract composition ME001 group (676.5 mg/kg B.W.), respectively. Five female mice were used in each group to perform this experiment. During the experimental period, with the exception of the mice in the normal control group which were fed with normal-fat diet, mice in other groups were fed with high-fat diet for 8 weeks consecutively to induce obesity. Simultaneously, experimental matters were administered by oral gavage daily for 8 weeks, while the same volume of sterile water was administered to the mice in the obese control group by oral gavage to evaluate the differences in weight and body fat among mice in the groups; meanwhile, body weight and average food intake of each animal were recorded weekly during the period of the experiment. After the assay has completed, the mice were sacrificed, and the fat around the ovary, the perinephric fat, and the mesenteric fat were dissected and weighed to calculate the amount of the visceral fat, and the fat around the groin and around the peritoneal cavity was dissected and weighed to calculate the amount of the subcutaneous fat. Data of each group are presented in Mean ± SD. The letters a, b, c, d, e, and f represent the results of the statistics; the different letters represent statistical difference among the groups (*p*<0.05), and the identical letter represents no statistical difference among the groups (*p*>0.05).

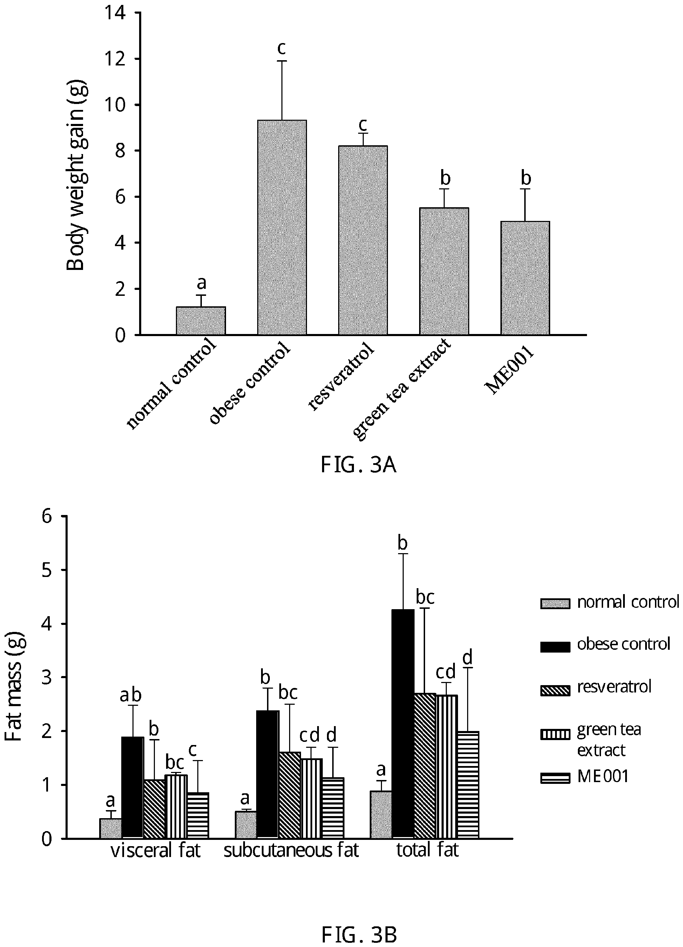

Results are shown in Fig. 3A and 3B, as compared with the obese control group, the total body weight gain of the mice in the plant extract composition ME001 group was significantly lower, wherein the reduction thereof was 47.2% (*p* <0.05). Therefore, the plant extract composition ME001 can achieve the effect of weight reduction effectively (*p* <0.05). In contrast, there is no significant difference in each of the total body weight gain, the amount of the visceral fat, the subcutaneous fat, and the total weight of the body fat (including the visceral fat and the subcutaneous fat) between the mice in the resveratrol group and the obese control group (*p*>0.05).

As compared to the obese control group, the plant extract composition ME001 could reduce the weight of the visceral fat, the subcutaneous fat, or the body fat significantly (*p* <0.05). Moreover, as compared to those of other groups, the plant extract composition ME001 can reduce the body weight and body fat effectively, and the effect was superior to those of the single plant extract groups (*p* <0.05); it had better weight loss effect.

During the period of the experiment, there was no statistical difference in the weekly average food intake among mice in all the groups which were fed with high-fat diet (*p>* 0.05).

### Example 4 Animal assay (II) (Administration after Obesity induction)

In this embodiment, 8-week-old female B6 mice were used. With the exception of the mice in normal control group which were fed with normal-fat diet, other mice were fed with high fat diet for six weeks consecutively to obtain obese mice (compared with the initial weight, the weight of the obese mice increased over 20%). The obese mice were divided into seven groups randomly, comprising an obese control group, an Orlistat (Xenical®) drug group (34.8 mg/kg B.W.), a turmeric extract group (41 mg/kg B.W.), and four plant extract composition experimental groups including ME008A (676.5 mg/kg B.W.), ME008D (676.5 mg/kg B.W.), ME001 (676.5 mg/kg B.W.), and ME00C1 (651.9 mg/kg B.W.). Five female mice were used in each group to perform this experiment. With the exception of the mice in the normal control group, obese mice in the other seven groups were fed with high fat diet and the experimental matters were simultaneously administered to these mice by oral gavage daily for weeks, while the same volume of sterile water were administered to the mice in the obese control group by oral gavage; after a total of 14 weeks of high fat diet feeding, the mice were sacrificed to evaluate the differences in body weight gain and body fat among mice in the groups; meanwhile, the body weight and the average food intake of each animal were recorded weekly during the period of the experiment. After the assay has completed, the mice were sacrificed, and the fat around the ovary, the perinephric fat, and the mesenteric fat were dissected and weighed to calculate the amount of the visceral fat, and the fat around the groin and around the peritoneal cavity was dissected and weighed to calculate the amount of the subcutaneous fat. Data of each group are presented in Mean ± SD. The letters a, b, c, d, and e represent the results of the statistics; the different letters represent statistical difference among the groups (*p*<0.05), and the identical letter represents no statistical difference among the groups (*p*>0.05).

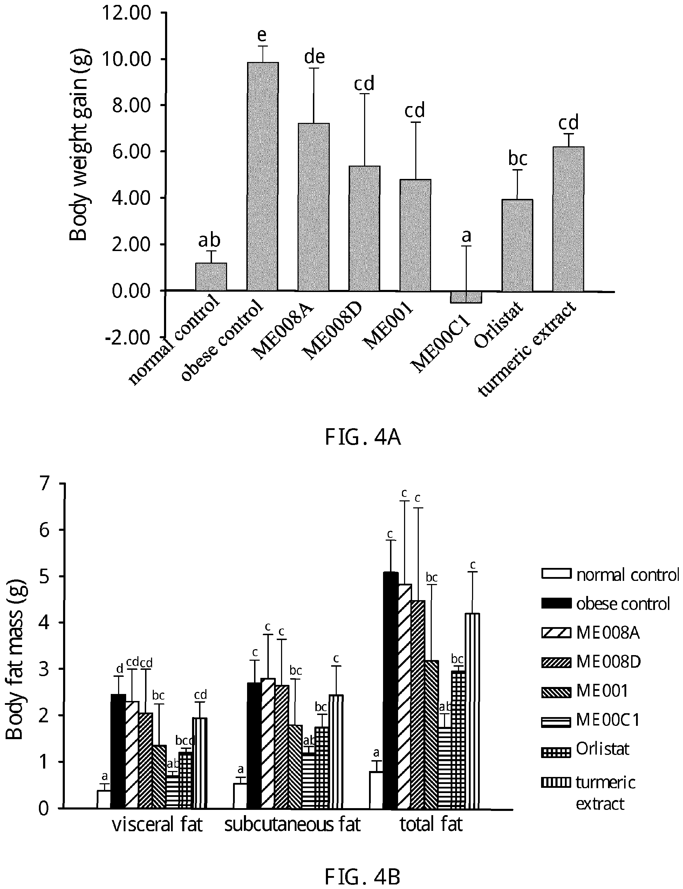

Results are shown in Fig. 4A and 4B, the weight gain and body fat of the mice in the obese control group was significantly higher than that of the normal control group, the weight gain was drastically increased as high as 87.7%, representing that the weight gain of the mice has been successfully induced to cause obesity in the present experiment. As compared to the obese control group, the plant extract compositions of ME008D and ME001 of the present disclosure, and ME00C1 of the present invention can reduce body weight gain significantly (*p* <0.05), wherein the plant extract composition ME00C1 of the present invention had the best reduction effect, and the effect is significantly better than that of the Orlistat, a commercially available weight loss drug, (*p* <0.05) and the turmeric extract (*p* <0.05).

As compared to the obese control group, the body fat (comprising visceral fat and subcutaneous fat) of mice in the groups of plant extract compositions ME008D and ME001 of the present disclosure, and ME00C1 of the present invention were reduced significantly (*p* <0.05), wherein the body fat was decreased by 10.3%, 36.9%, and 64.1% respectively. The plant extract composition MEOOC1 of the present invention had the best effect, and the effect is significantly better than those of the Orlistat, a commercially available weight loss drug, (*p* <0.05) and the turmeric extract (*p* <0.05). It indicates that the plant extract composition ME00C1 of the present invention has a better weight loss effect and body fat reduction effect on obese mice. During the period of the experiment, there was no statistical difference in the weekly average food intake among mice in all of the groups which were fed with high-fat diet (*p>* 0.05).

### Example 5 Animal assay (III) (Administration during obesity induction)

In this embodiment, 8-week-old male Sprague-Dawley (SD) rats were used. There were four groups in this experiment which were a normal control group, an obese control group, plant extract composition ME00C1 group (199.6 mg/kg B.W.) and plant extract composition ME00C1A group (186 mg/kg B.W.) of the present invention, wherein the composition ME00C1A contained 55.5 wt% green tea extract and 44.5 wt% turmeric extract. Six rats were used in each group to perform this experiment. During the experimental period, with the exception of the normal control group which were fed with normal diet, rats in the remaining three groups were continuously fed with high-fat diet for 8 weeks to induce obesity. Simultaneously, experimental matters were administered by oral gavage daily, while the same volume of sterile water were administered to the rats in the obese control group to evaluate the differences in the weight gain and the visceral fat among mice in the groups. During the period of the experiment, the body weight and the average food intake of each animal were recorded weekly. After the assay has completed, the rats were sacrificed, and the epididymal fat, the perinephric fat, and the mesenteric fat were dissected and weighed to calculate the amount of the visceral fat. Data of each group are presented in Mean ± SD. The letters a, b, c, and d represent the results of the statistics; the different letters represent statistical difference among the groups (*p*<0.05), and the identical letter represents no statistical difference among the groups (*p*>0.05).

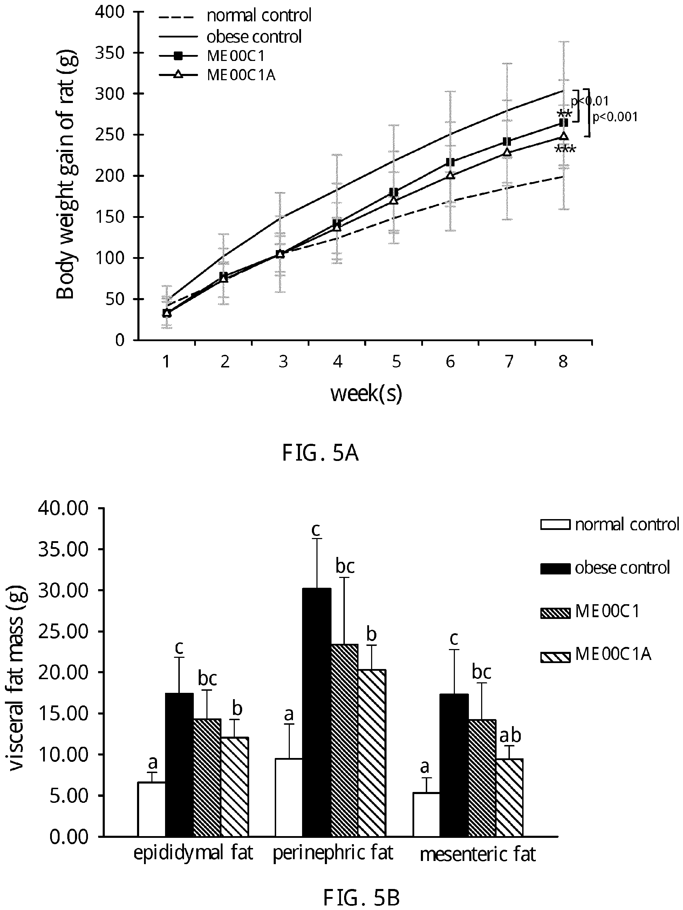

As shown in Fig. 5A, the body weight gain of the rats, to which the plant extract compositions ME00C1 or ME00C1A of the present invention were administered, were decreased significantly compared to that of the rats in the obese control group. The body weight gain of the rats in the plant extract composition ME00C1 group were decreased significantly by 23.0% (*p*<0.01 t-test), and the body weight gain of the rats in the plant extract composition ME00C1A group could even be significantly decreased by 29.8% (*p*<0.01 valued by t-test). Experimental results shown in Fig. 5B indicate that the visceral fat such as epididymal fat, perinephric fat, and mesenteric fat of the rats in the plant extract composition ME00C1A group of the present invention was reduced significantly (*p* <0.05) compared to the obese control group, wherein the total weight of the visceral fat could decreased as much as 35.7%. It indicates that the plant extract composition ME00C1A of the present invention had the best reduction effect on body weight gain and body fat.

The above descriptions are merely the preferred embodiments, and are not limitation to the present invention in any form; even though the preferred embodiments of the present invention are disclosed above, it is not used to limit the present invention by any means.

## Claims

1. A composition comprising a green tea extract and a turmeric extract for use in a method of treating obesity, wherein the weight percentages of green tea extract and turmeric extract are respectively 30 % to 75 % and 20 % to 55 % based on the total weight of the composition, wherein the green tea extract comprises 75% to 100% catechins and the turmeric extract comprises 80% to 100% curcumin, and wherein the method comprises administration of the composition to a subject such that body weight or body fat of the subject is reduced.

2. The composition for the use of claim 1, wherein the weight percentages of green tea extract and turmeric extract are respectively 55.5 % to 75 % and 25 % to 44.5 % based on the total weight of the composition.

3. The composition for the use of claim 1 or 2, wherein the composition is a pharmaceutical composition and further comprises a pharmaceutically acceptable excipient.

4. The composition for the use of claim 3, wherein the subject is a human or an animal.

5. The composition for the use of claim 4, wherein the method comprises administration of the composition by oral administration or injection.

6. The composition for the use of claim 5, wherein the amount of the composition administered to the subject by oral administration or injection is from 1.8 mg/kg to 145 mg/kg.

7. The composition for the use of claim 6, wherein the amount of the composition administered to the subject by oral administration or injection is from 5.4 mg/kg to 70 mg/kg.

8. The composition for the use of any of claims 1 to 7, wherein the composition further comprises resveratrol, and the weight percentages of resveratrol is more than 0 % and is less than or equal to 30 %.

9. Use of a composition for non-therapeutic reduction of body weight or body fat of a subject, wherein the composition comprises a green tea extract and a turmeric extract, the weight percentages of green tea extract and turmeric extract are respectively 30 % to 75 % and 20 % to 55 % based on the total weight of the composition, wherein the green tea extract comprises 75% to 100% catechins and the turmeric extract comprises 80% to 100% curcumin, and wherein the composition is to be administered to the subject such that body weight or body fat of the subject is reduced.

10. The use of claim 9, wherein the weight percentages of green tea extract and turmeric extract are respectively 55.5 % to 75 % and 25 % to 44.5 % based on the total weight of the composition.

11. The use of claim 8 or 9, wherein the composition is a pharmaceutical composition and further comprises a pharmaceutically acceptable excipient.

12. The use of claim 11, wherein the subject is a human or an animal.

13. The use of claim 12, wherein the composition is administered by oral administration or injection.

14. The use of claim 13, wherein the amount of the composition administered to the subject by oral administration or injection is from 1.8 mg/kg to 145 mg/kg.

15. The use of claim 14, wherein the amount of the composition administered to the subject by oral administration or injection is from 5.4 mg/kg to 70 mg/kg.

16. The use of any of claims 9-15, wherein the composition further comprises resveratrol, and the weight percentages of resveratrol is more than 0 % and is less than or equal to 30 %.

## Patentansprüche

1. Zusammensetzung, umfassend einen Grüntee-Extrakt und einen Kurkuma-Extrakt zur Verwendung in einem Verfahren zur Behandlung von Adipositas, wobei die prozentualen Gewichtsanteile von Grüntee-Extrakt und Kurkuma-Extrakt 30 % bis 75 % bzw. 20 % bis 55 %, bezogen auf das Gesamtgewicht der Zusammensetzung, betragen, wobei der Grüntee-Extrakt 75 % bis 100 % Katechine und der Kurkuma-Extrakt 80 % bis 100 % Curcumin enthält, und wobei das Verfahren die Verabreichung der Zusammensetzung an ein Subjekt umfasst, so dass das Körpergewicht oder Körperfett des Subjekts reduziert wird.

2. Zusammensetzung zur Verwendung von Anspruch 1, wobei die prozentualen Gewichtsanteile von Grüntee-Extrakt und Kurkuma-Extrakt 55,5 % bis 75 % bzw. 25 % bis 44,5 %, bezogen auf das Gesamtgewicht der Zusammensetzung, betragen.

3. Zusammensetzung zur Verwendung von Anspruch 1 oder 2, wobei die Zusammensetzung eine pharmazeutische Zusammensetzung ist und ferner einen pharmazeutisch verträglichen Hilfsstoff enthält.

4. Zusammensetzung zur Verwendung von Anspruch 3, wobei das Subjekt ein Mensch oder ein Tier ist.

5. Zusammensetzung zur Verwendung von Anspruch 4, wobei das Verfahren die Verabreichung der Zusammensetzung durch orale Verabreichung oder Injektion umfasst.

6. Zusammensetzung zur Verwendung von Anspruch 5, wobei die Menge der Zusammensetzung, die dem Subjekt durch orale Verabreichung oder Injektion verabreicht wird, von 1,8 mg/kg bis 145 mg/kg beträgt.

7. Zusammensetzung zur Verwendung von Anspruch 6, wobei die Menge der Zusammensetzung, die dem Subjekt durch orale Verabreichung oder Injektion verabreicht wird, von 5,4 mg/kg bis 70 mg/kg beträgt.

8. Zusammensetzung zur Verwendung nach einem der Ansprüche 1 bis 7, wobei die Zusammensetzung ferner Resveratrol umfasst und die prozentualen Gewichtsanteile von Resveratrol mehr als 0 % und weniger als oder gleich 30 % betragen.

9. Verwendung einer Zusammensetzung zur nicht-therapeutischen Reduktion des Körpergewichts oder des Körperfetts eines Subjekts, wobei die Zusammensetzung einen Grüntee-Extrakt und einen Kurkuma-Extrakt umfasst, wobei die prozentualen Gewichtsanteile des Grüntee-Extrakts und des Kurkuma-Extrakts 30 % bis 75 % bzw. 20 % bis 55 %, bezogen auf das Gesamtgewicht der Zusammensetzung, betragen, wobei der Grüntee-Extrakt 75 % bis 100 % Katechine und der Kurkuma--Extrakt 80 % bis 100 % Curcumin enthält, und wobei die Zusammensetzung dem Subjekt so zu verabreichen ist, dass das Körpergewicht oder Körperfett des Subjekts reduziert wird.

10. Verwendung nach Anspruch 9, wobei die prozentualen Gewichtsanteile von Grüntee-Extrakt und Kurkuma-Extrakt 55,5 % bis 75 % bzw. 25 % bis 44,5 %, bezogen auf das Gesamtgewicht der Zusammensetzung, betragen.

11. Verwendung nach Anspruch 8 oder 9, wobei die Zusammensetzung eine pharmazeutische Zusammensetzung ist und ferner einen pharmazeutisch verträglichen Hilfsstoff enthält.

12. Verwendung nach Anspruch 11, wobei das Subjekt ein Mensch oder ein Tier ist.

13. Verwendung nach Anspruch 12, wobei die Zusammensetzung durch orale Verabreichung oder Injektion verabreicht wird.

14. Verwendung nach Anspruch 13, wobei die Menge der Zusammensetzung, die dem Subjekt durch orale Verabreichung oder Injektion verabreicht wird, von 1,8 mg/kg bis 145 mg/kg beträgt.

15. Verwendung nach Anspruch 14, wobei die Menge der Zusammensetzung, die dem Subjekt durch orale Verabreichung oder Injektion verabreicht wird, von 5,4 mg/kg bis 70 mg/kg beträgt.

16. Verwendung nach einem der Ansprüche 9-15, wobei die Zusammensetzung ferner Resveratrol umfasst und die prozentualen Gewichtsanteile von Resveratrol mehr als 0 % und weniger als oder gleich 30 % betragen.

## Revendications

1. Une composition comprenant un extrait de thé vert et un extrait de curcuma pour utilisation dans un procédé de traitement de l'obésité, dans laquelle les pourcentages en poids d'extrait de thé vert et d'extrait de curcuma sont respectivement de 30 % à 75 % et de 20 % à 55 % sur la base du poids total de la composition, dans laquelle l'extrait de thé vert comprend 75 % à 100 % de catéchines et l'extrait de curcuma comprend 80 % à 100 % de curcumine, et dans laquelle le procédé comprend l'administration de la composition à un sujet de telle sorte que le poids corporel ou la graisse corporelle du sujet est réduit.

2. La composition pour l'utilisation selon la revendication 1, dans laquelle les pourcentages en poids d'extrait de thé vert et d'extrait de curcuma sont respectivement de 55,5 % à 75 % et de 25 % à 44,5 % sur la base du poids total de la composition.

3. La composition pour l'utilisation selon la revendication 1 ou 2, dans laquelle la composition est une composition pharmaceutique et comprend en outre un excipient pharmaceutiquement acceptable.

4. La composition pour l'utilisation selon la revendication 3, dans laquelle le sujet est un être humain ou un animal.

5. La composition pour l'utilisation selon la revendication 4, dans laquelle le procédé comprend l'administration de la composition par administration orale ou par injection.

6. La composition pour l'utilisation selon la revendication 5, dans laquelle la quantité de la composition administrée au sujet par administration orale ou par injection est de 1,8 mg/kg à 145 mg/kg.

7. La composition pour l'utilisation selon la revendication 6, dans laquelle la quantité de la composition administrée au sujet par administration orale ou par injection est de 5,4 mg/kg à 70 mg/kg.

8. La composition pour l'utilisation selon l'une quelconque des revendications 1 à 7, dans laquelle la composition comprend en outre du resvératrol, et les pourcentages en poids de resvératrol sont supérieurs à 0 % et inférieurs ou égaux à 30 %.

9. Utilisation d'une composition pour la réduction non thérapeutique du poids corporel ou de la graisse corporelle d'un sujet, dans laquelle la composition comprend un extrait de thé vert et un extrait de curcuma, les pourcentages en poids d'extrait de thé vert et d'extrait de curcuma sont respectivement de 30 % à 75 % et de 20 % à 55 % sur la base du poids total de la composition, dans laquelle l'extrait de thé vert comprend 75 % à 100 % de catéchines et l'extrait de curcuma comprend 80 % à 100 % de curcumine, et dans laquelle la composition doit être administrée au sujet de telle sorte que le poids corporel ou la graisse corporelle du sujet est réduit.

10. L'utilisation selon la revendication 9, dans laquelle les pourcentages en poids d'extrait de thé vert et d'extrait de curcuma sont respectivement de 55,5 % à 75 % et de 25 % à 44,5 % sur la base du poids total de la composition.

11. L'utilisation selon la revendication 8 ou 9, dans laquelle la composition est une composition pharmaceutique et comprend en outre un excipient pharmaceutiquement acceptable.

12. L'utilisation selon la revendication 11, dans laquelle le sujet est un être humain ou un animal.

13. L'utilisation selon la revendication 12, dans laquelle la composition est administrée par administration orale ou par injection.

14. L'utilisation selon la revendication 13, dans laquelle la quantité de la composition administrée au sujet par administration orale ou par injection est de 1,8 mg/kg à 145 mg/kg.

15. L'utilisation selon la revendication 14, dans laquelle la quantité de la composition administrée au sujet par administration orale ou par injection est de 5,4 mg/kg à 70 mg/kg.

16. L'utilisation selon l'une quelconque des revendications 9 à 15, dans laquelle la composition comprend en outre du resvératrol, et les pourcentages en poids de resvératrol sont supérieurs à 0 % et inférieurs ou égaux à 30 %.
